# EUROPEAN PATENT SPECIFICATION

(11) **EP 2 305 654 B1**
(45) Date of publication and mention of the grant of the patent: **14.10.2015**
(21) Application number: 09746375.6
(22) Date of filing: 14.05.2009
(51) Int. Cl.: C07D 243/12, A61K 31/551, A61K 45/00, A61P 25/04

(54) **THERAPEUTIC AGENT FOR CANCER PAIN**
THERAPEUTISCHES MITTEL GEGEN KREBSSCHMERZEN
AGENT THÉRAPEUTIQUE POUR LA DOULEUR DUE À UN CANCER

(30) Priority: 15.05.2008 JP 2008128735
(43) Date of publication of application: 06.04.2011
(73) Proprietor: Zeria Pharmaceutical Co., Ltd., Tokyo 103-8351 (JP)
(72) Inventor: YOSHINAGA, Koji, Tokyo 103-8351 (JP); HAMANO, Hiroki, Tokyo 103-8351 (JP); HORII, Takayuki, Tokyo 103-8351 (JP)
(74) Representative: Hartz, Nikolai
(86) International application number: PCT/JP2009/002109
(87) International publication number: WO 2009/139168

(56) References cited:
- EP-A1- 0 945 445
- EP-A1- 1 234 818
- EP-A1- 1 839 662
- US-A- 5 484 917
- XIAO-JUN X ET AL: "Chronic pain-related behaviors in spinally injured rats: Evidence for functional alterations of the endogenous cholecystokinin and opioid systems", PAIN, ELSEVIER SCIENCE PUBLISHERS, AMSTERDAM, NL, vol. 56, no. 3, 1 March 1994 (1994-03-01), pages 271-277, XP024377827, ISSN: 0304-3959, DOI: 10.1016/0304-3959(94)90165-1 [retrieved on 1994-03-01]
- KAWASAKI, D. ET AL.: 'Effect of Z-360, a novel orally active CCK-2/gastrin receptor antagonist on tumor growth in human pancreatic adenocarcinoma cell lines in vivo and mode of action determinations in vitro' CANCER CHEMOTHERAPY AND PHARMACOLOGY vol. 61, no. 5, April 2008, pages 883 - 892, XP019587410
- GRABOWSKA, A.M. ET AL.: 'Pre-clinical evaluation of a new orally-active CCK-2R antagonist, Z-360, in gastrointestinal cancer models' REGULATORY PEPTIDES vol. 146, no. 1-3, February 2008, pages 46 - 57, XP022421983
- HRUBY, V.J. ET AL.: 'New paradigms and tools in drug design for pain and addiction' AAPS JOURNAL vol. 8, no. 3, 2006, pages E450 - E460, XP008140865
- YENNURAJALINGAM, S. ET AL.: 'Recent developments in cancer pain assessment and management' SUPPORTIVE CANCER THERAPY vol. 1, no. 2, 2004, pages 97 - 110, XP008140863

## Description

### Technical Field

The present invention related to a therapeutic or prophylactic agent for use in the treatment or prevention of pain attributable to cancer (hereinafter referred to as cancer pain).

### Background Art

Xiao-Jun X. et al., Pain, vol.56, no.3, pages 271-277 (1994), discloses effects of VI988 on allodynia. EP-A 1 234 818 discloses 1,5-benzodiazepine derivatives having inhibitory activity against the secretion of gastric acid and antagonism against gastrin and/or CCK-B receptor. EP-A 0 945 445 dislcoses 1,5-benzodiazepine derivatives having gastrin and/or CCK-B receptor antagonism as remedies for gastric ulcer and gastrointestinal movement disorder.

Pain is a result of sensing of physical stimulation or chemical stimulation, by a pain-causing substance by the sensory nerve ending plate, and recognition of the stimulation as pain by the cerebrum. "Pain" is one of the most undesired factors that impair QOL. Pain is generally categorized in terms of the origin to three: nociceptive pain, neuropathic pain, and psychogenic pain. Nociceptive pain occurs by the mediation of nociceptors when a tissue is damaged or noxious stimulation that may damage a tissue is applied to the living body. Neuropathic pain is caused by primary damage of the nervous system or a function disorder of the nervous system or induced by the damage or disorder. Neuropathic pain is caused by damage of the peripheral nervous system or the central nervous system. Psychogenic pain is a type of pain which exhibits no lesion sufficient to cause pain and which cannot be elucidated anatomically.

Typical examples of nociceptive pain include muscle pain, joint pain, headache, oral/facial pain, and visceral pain. Examples of neuropathic pain include pain in diabetes patients and in alcoholic patients, adverse side effects of anticancer agents (e.g., cisplatin, paclitaxel, vincristine), postoperative pain, phantom pain, postherpetic pain, trigeminal neuralgia, and central neuralgia.

It has been reported that cancer pain occurs in 30% of early-stage patients and 70% of final-stage patients (Non-Patent Document 1). Cancer pain is a type of complex pain including nociceptive pain and neuropathic pain among the above three categories and is caused by the following complex factors.

In the early stage, pain mainly occurs by cancer lesions (e.g., pain caused by bone metastasis, nerve compression, or impaired blood flow, cancer infiltration to the visceral organs, and brain metastasis). In the progress of the disease, pain relating to general hyposthenia (decubitus, muscle convulsion, and constipation) occurs. In addition, allodynia (i.e., pain caused by a stimulus that generally induces no pain) also onsets. Therefore, pain control of cancer patients is an important factor in cancer therapy.

The World Health Organization (WHO) has established a worldwide standard for pain treatment employing an opioid analgesic agent (Non-Patent Document 2). Currently, a therapeutic method in accordance with the standard is employed as the mainstream therapy for cancer pain.

The above method employs a drug administration regimen in which the drug is changed in three steps: a non-opioid analgesic agent, a weak opioid analgesic agent, and a strong opioid analgesic agent. If required, an additional analgesic adjuvant such as an antidepressant, an anticonvulsant, a local anesthetic, a glucocorticoid, a psychotropic agent, or an antihistamic agent is administered in combination.

Examples of non-opioid analgesic agents which are mainly employed include NSAID and acetaminophen. Examples of weak opioid analgesic agents employed include codeine, and examples of opioid analgesic agents employed include morphine, methadone, pethidine, buprenorphine, hydromorphine, levorphanol, oxycodone, and fentanyl.

Among non-opioid analgesic agents, NSAID is known to often cause gastrointestinal disorders and renal disorders, which are adverse side effects of the agent. Among the opioid analgesic agents, morphine provides adverse side effects generally including constipation, nausea, and vomiting. If administration is suddenly stopped or the dose is suddenly reduced, morphine is known to cause a withdrawal symptom (Non-Patent Document 3).

Meanwhile, cancer pain includes neuropathic pain, which is generated by damage of the peripheral nerve or the central nerve. Therefore, opioid often fails to exert the effects thereof (opioid resistance). During chronic administration of morphine, the effect of an anti-opioid substance such as cholecystokinin (CCK) or neuropeptide Y (NPY) is potentiated, thereby strongly inhibiting the analgesia by morphine. In this case, the analgesic effect of morphine is reduced, to thereby provide analgesic resistance (Non-Patent Document 3), which is a big impediment in pain control.

At present, analgesic adjuvants which inhibit neurotransmission such as an anticonvulsant and an antidepressant are used in combination. Although remarkable effects are not attained, these analgesic adjuvants are expected to be effective for paroxysmal pain and a state of depression caused by pain (Non-Patent Document 3).

However, the aforementioned therapeutic effects are unsatisfactory, and thus, there is demand for the development of an analgesic agent with less adverse side effects than currently employed non-opioid analgesic agents, opioids, and analgesic adjuvants.

As described above, generally, the analgesic effect of morphine is partially antagonized by an anti-opioid substance. Thus, an anti-opioid substance is thought to be involved in a mechanism of morphine resistance and morphine dependence. Therefore, morphine resistance is thought to be suppressed by use of a substance which antagonizes an anti-opioid.

In one study, a CCK2 receptor antagonist L-365,260 having central transferability (a benzodiazepine compound) has been reported to suppress generation of morphine resistance in a rat neuropathic pain model (Non-Patent Document 4). It has been also reported that L-365,260 and CI-988 (C-terminal pentapeptide derivative of CCK, known as potent CCK2 receptor antagonist) suppress generation of morphine resistance in a mouse nociceptive pain model (pain caused by thermal stimulation) (Non-Patent Documents 5 and 6).

In a rat neuro-damaged model, L-365,260 potentiates the analgesic effect of morphine on nociceptive pain caused by thermal stimulation (Non-Patent Document 7). CI-988 potentiates the analgesic effect of morphine on nociceptive pain of mice chemically triggered by formalin (Non-Patent Document 8). In a rat neuropathic pain model, L-365,260 potentiates the analgesic effect of morphine on allodynia (Non-Patent Document 9).

In some clinical tests, proglumide, which is a CCK2 receptor antagonist, potentiates the analgesic effect of morphine on cancer pain (Non-Patent Document 10).

As described above, the analgesic effect of morphine is known to be generally potentiated by a CCK2 receptor antagonist.

Meanwhile, there are various opinions about the analgesic effect of a CCK2 receptor antagonist in sole use, and it has not been elucidated whether or not a CCK2 receptor antagonist exhibits analgesic effect when administered singly.

One study has reported that L-365,260 or proglumide, in sole use, exhibits the analgesic effect on nociceptive pain of mice chemically triggered by formalin (Non-Patent Document 11). There has been reported an analgesic effect of CCK2 receptor antagonist YM022 in sole use on thermal hyperalgesia developed in a rat neuropathic pain model (Non-Patent Document 12).

On the other hand, in a rat neuro-damaged model, single use of L-365,260 exhibits no analgesic effect on nociceptive pain caused by thermal stimulation (Non-Patent Document 7). Single use of L-365,260 exhibits no analgesic effect on nociceptive pain of mice caused by thermal stimulation (Non-Patent Document 13). Furthermore, single use of L-365,260 exhibits no analgesic effect on a rat neuropathic pain model (Non-Patent Document 9).

As described above, development of pharmaceutical products for the cancer pain therapy involves complicated factors. Thus, production of a cancer pain animal model by use of malignant tumor cells and analysis of the model are thought to be important (Non-Patent Document 14). Is has not been known what kind of analgesic effect the CCK2 receptor antagonists hitherto developed exhibits on the cancer pain model (in an animal experiment). Furthermore, no clinical report has been stated that sole use of a CCK2 receptor antagonist exhibits analgesic effect on cancer pain.

As mentioned above, a variety of CCK2 receptor antagonists have been already developed, but there are various opinions about the analgesic effects of these antagonists. That is, the relationship between CCK2 receptor antagonism and analgesic effect is not considered to be a simple relationship, and the role of a CCK2 receptor has not been completely elucidated. In addition, it has not been clearly elucidated whether or not the analgesic effect of a substance having CCK2 receptor antagonism which has already been reported is attributed to the CCK2 receptor.

1,5-Benzodiazepine compounds disclosed in Patent Document 1 are known to have CCK2 receptor antagonism. However, whether or not the compounds have useful analgesic effect is unknown.

Meanwhile, the tumor regression effect of an anticancer agent and the pain relief effect thereof are not always correlated with each other. Actually, gemcitabine hydrochloride is used in the treatment of pancreatic cancer, which is known to give severe pain to the patients thereof, which pain is very difficult to control. Among cancer chemotherapeutic agents, gemcitabine hydrochloride is known to have comparatively weak tumor regression effect but to exhibit excellent pain relief effect on pancreatic cancer patients (Non-Patent Document 15). As compared with gemcitabine hydrochloride, use in combination of irinotecan and gemcitabine hydrochloride provides potent tumor regression effect on pancreatic cancer. However, no difference is observed in terms of QOL evaluation including pain relief, and survival period is rather shortened (Non-Patent Document 16). Thus, whether or not a drug exhibiting an antitumor effect is always useful for pain relief has not been elucidated.

### Related Art Documents

### Patent Documents

Patent Document 1: WO 98/25911
Patent Document 2: WO 01/40197
Patent Document 3: WO 2006/077793

### Non-Patent Documents

Non-Patent Document 1: Foley KM, Arch Neurol 1999, 56, 413-417
Non-Patent Document 2: WHO Cancer Pain Relief, 2nd edition 1996
Non-Patent Document 3: Easy Algesiology, Ueda et al., 2007, Brain-shuppan
Non-Patent Document 4: Idanpaan-Heikkila J. J. et al., J. Pharma. Exper. Ther 1997, 282, 3, 1366-72
Non-Patent Document 5: Zarrindast M. R. et al., Pharmacol. Biochem. Behav. 1997, 58, 1, 173-8
Non-Patent Document 6: Xu X. J. et al., Br. J. Pharmacol. 1992, 105, 591-96
Non-Patent Document 7: Idanpaan-Heikkila J. J. et al., Eur. J. Pharmacol. 1997, 325, 155-64
Non-Patent Document 8: Nobel F. et al., Eur. J. Pharmacol. 1995, 273, 145-51
Non-Patent Document 9: Nichols M. L. et al., J. Pharma. Experi. Ther. 1995, 275, 3, 1339-45
Non-Patent Document 10: Bernstein Z. P. et al., J. Pain Symptom Management, 1998, 15, 5, 314-20
Non-Patent Document 11: Rezayat M. et al., Eur. Neuropsychopharmacolgy 1999, 9, 9-14
Non-Patent Document 12: Yamamoto T. et al., Neuroscience Lett. 1995, 202, 89-92
Non-Patent Document 13: Vanderah T. W. et al., J. Pharma. Experi. Ther. 1996, 278, 1, 212-9
Non-Patent Document 14: Kuraishi et al., Journal of Clinical and Experimental Medicine (Igaku-no-Ayumi) 2007,223,9,736-741
Non-Patent Document 15: Burris H. A. et al., J. Clin. Oncol. 1997, 15(6), 2403-13
Non-Patent Document 16: Rocha Lima C. M. et al., J. Clin. Oncol. 2004, 22(18), 3776-83

### Summary of the Invention

### Problems to be Solved by the Invention

An object of the present invention is to provide a cancer pain therapeutic agent.

### Means for Solving the Problems

The present inventors have conducted extensive studies on the effect of a CCK2 receptor antagonist on pain therapy, and have found that, quite surprisingly, a 1,5-benzodiazepine derivative or a pharmaceutically acceptable salt thereof disclosed in WO 01/40197 exhibits excellent cancer pain therapeutic effect, although L-365,260 or proglumide, which is a known CCK2 receptor antagonist, exhibits no therapeutic effect on cancer pain. The inventors have also found that the cancer pain therapeutic effect of the 1,5-benzodiazepine derivative is remarkably enhanced when the derivative is used in combination with another analgesic agent such as morphine.

Accordingly, the present invention provides a 1,5-benzodiazepine derivative represented by formula (1): 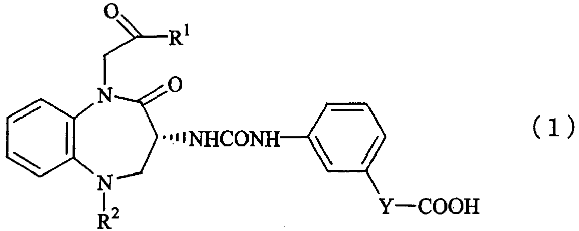 (wherein R¹ represents a C₁₋₆ alkyl group, R² represents a phenyl group or a cyclohexyl group, and Y represents a single bond or a C₁₋₄ an alkylene group) or a pharmaceutically acceptable salt thereof, for use in the treatment or prevention of cancer pain at a peroral daily dose for an adult of 50 to 600 mg.

The use of a 1,5-benzodiazepine derivative represented by formula (1) or a pharmaceutically acceptable salt thereof, for producing a cancer pain therapeutic and/or prophylactic agent is disclosed.

A method for treatment of cancer pain, comprising administering, to a subject in need thereof, an effective amount of a 1,5-benzodiazepine derivative represented by formula (1) or a pharmaceutically acceptable salt thereof is also disclosed.

A cancer pain therapeutic and/or prophylactic agent, comprising, in combination, a 1,5-benzodiazepine derivative represented by formula (1) or a pharmaceutically acceptable salt thereof and another analgesic agent, is also disclosed.

### Effects of the Invention

The compound for use in the present invention has no severe adverse side effect in a safety test employing an animal. Thus, the compound for use in the invention can be administered to a patient for a long period of time, without causing an adverse side effect which a conventional non-opioid analgesic agent or opioid analgesic agent has. Therefore, the compound for use in the invention can be advantageously administered as a pain therapeutic agent in the treatment of cancer pain from the initial stage to the final stage.

In addition, the pharmaceutical for use in the present invention, having low toxicity, can be administered continuously and perorally, thereby providing a simple dosage form. Non-Patent Document 2 discloses that peroral administration of an analgesic agent is an essential principle in cancer pain treatment. That is, if a drug can be perorally administered, no particular complex apparatus is required, and pain treatment can be satisfactorily performed in a patient's home, which would be very advantageous to the patient.

Most known CCK2 receptor antagonists exhibit virtually no cancer pain therapeutic effect, whereas the compound of the present invention exhibits excellent cancer pain therapeutic effect. Therefore, the pain therapeutic effect of the compound for use in the present invention is thought to be not based on CCK2 receptor antagonism. No correlation was found between the pain therapeutic effect of the compound for use in the present invention and antitumor effect thereof.

### Brief Description of the Drawings

[Fig. 1] A graph showing the cancer pain therapeutic effect of single administration of compound (A1).
[Fig. 2] A graph showing the cancer pain therapeutic effect of continuous administration of compound (A1).
[Fig. 3] A graph showing the cancer pain therapeutic effect of compound (A1) and morphine in combination.

### Modes for Carrying Out the Invention

In formula (1), examples of the C₁₋₆ alkyl group represented by R¹ include a methyl group, an ethyl group, a propyl group, an isopropyl group, a butyl group, an isobutyl group, a sec-butyl group, and a tert-butyl group. Of these, a C₁₋₄ alkyl group is preferred, with a C₄ alkyl group being more preferred, and a tert-butyl group being particularly preferred.

The group R² is particularly preferably a cyclohexyl group. Examples of the C₁₋₄ alkylene group represented by Y include a methylene group, an ethylene group, a propylene group, a butylene group, a methylmethylene group, a dimethylmethylene group, a 1-methylethylene group, a 1,1-dimethylethylene group, a 1-methylpropylene group, and a 2-methylpropylene group. Of these, a dimethylmethylene group is particularly preferred. Y is particularly preferably a single bond.

Among compounds (1), particularly preferred are (R)-(-)-3-[3-(1-tert-butylcarbonylmethyl-2-oxo-5-cyclohexyl-1,3,4,5-tetrahydro-2H-1,5-benzodiazepin-3-yl)ureido]beozoic acid or a pharmaceutically acceptable salt thereof (compound A), and (R)-(-)-2-[3-[3-(1-tert-butylcarbonylmethyl-2-oxo-5-cyclohexyl-1,3,4,5-tetrahydro-2H-1,5-benzodiazepin-3-yl)ureido]phenyl-2-methylpropionic acid or a pharmaceutically acceptable salt thereof (compound B). Of these, compound A is particularly preferred.

Examples of the salt of compound (1) include inorganic salts such as sodium salts, potassium salts, calcium salts, and magnesium salts; organic salts such as ammonium salts, pyridine salts, triethylamine salts, ethanolamine salts, (R) or (S) α-phenethylamine salts, benzylamine salts, and 4-methylbenzylamine salts; and acid addition salts with organic or inorganic acid. Of these, basic salts are preferred, and inorganic basic salts are more preferred. Among inorganic salts, alkaline earth metal salts, particularly calcium salts, are preferred.

The compound (1) encompasses optically active forms and diastereomers as well as solvates (e.g., hydrates) and crystal polymorphisms.

The compound (1) may be produced through a method disclosed in WO 01/40197.

As described in the Examples hereinbelow, the compound (1) mitigates allodynia, a type of cancer pain, when used as a single agent. Therefore, the compound (1) is a useful cancer pain therapeutic and/or prophylactic agent to various cancers. No particular limitation is imposed on the target cancer to which the cancer pain therapeutic and/or prophylactic agent is applied. Examples of the target cancer include brain tumor, breast cancer, endometrial cancer, cervical cancer, ovarian cancer, stomach cancer, appendiceal cancer, colorectal cancer, liver cancer, gallbladder cancer, bile duct cancer, pancreatic cancer, gastrointestinal tract interstitial tumor, mesothelioma, head and neck cancer, kidney cancer, lung cancer, osteosarcoma, prostate cancer, testicular tumor, kidney cancer, bladder cancer, rhabdomyosarcoma, skin cancer, leukemia, lymphoma, and multiple myeloma. The compound (1) may be used not only as a cancer pain therapeutic agent but also as another analgesic agent.

The cancer pain therapeutic and/or prophylactic agent for use in the present invention may be mixed with a pharmaceutically acceptable carrier or aid, and the mixture may be orally administered. Examples of the dosage form of oral administration include solid preparations such as tablets, granules, powder, and capsules. Solid preparations may further contain an appropriate additive, and examples of the additive include excipients such as lactose, mannit, corn starch, and crystalline cellulose; binders such as a cellulose derivative, gum arabic, and gelatin; disintegrators such as carboxymethylcellulose calcium; and lubricants such as talc and magnesium stearate. The solid preparations may be controlled release preparations, which are formed by use of a coating material such as hydroxymethylcellulose phthalate, hydroxypropylmethylcellulose acetate succinate, cellulose acetate phthalate, or methacrylate copolymer. The compund (1) may be formed into a liquid preparation such as liquid, suspension, or emulsion.

For parenteral dosage forms, an injection may be provided. In this case, a generally employed surfactant such as water, ethanol, or glycerin may be incorporated thereinto. Similarly, a suppository may be formed by use of an appropriate base.

The dose of compound (1) contained in the cancer pain therapeutic and/or prophylactic agent of the present invention is appropriately determined in accordance with the conditions of each case in consideration of administration route; dosage form; the condition, age, sex, etc., of the patient; etc. The peroral daily dose for an adult is 50 to 600 mg, preferably 180 to 500 mg. The compound (1) is preferably administered once a day or in a divided manner of 2 to 3 times a day.

Needless to say, the cancer pain therapeutic and/or prophylactic agent of the present invention may be used singly. However, the agent for use in the invention may be administered in combination with at least one member of other non-opioid analgesic agents and opioid analgesic agents. The timing, frequency, and route of these ingredients may be identical to or different from one another. The dose of compound (1) and the dose of another cancer pain therapeutic and/or prophylactic agent used in combination are appropriately determined drug by drug in accordance with the type of agent used in combination, the condition of patients, administration route, etc. Through combination of compound (1) with such agents, pain of cancer patients is mitigated, whereby QOL of the patients can be improved.

The analgesic agent which may be used in combination with compound (1) is preferably an opioid analgesic agent, particularly preferably morphine, methadone, pethidine, buprenorphine, hydromorphine, levorphanol, oxycodone, or fentanyl.

The compound (1) and another analgesic agent may be administered simultaneously or separately. The administration routes of the agents may be different from one another.

As shown in the Examples hereinbelow, being different from comparative substances having CCK2 antagonism, the cancer pain therapeutic and/or prophylactic agent for use in the present invention exhibits cancer pain relief effect by sole use thereof without using morphine in combination. Thus, the agent of the invention can be used as a novel pain therapeutic agent in the treatment of cancer pain. Furthermore, when compound (1) is used in combination with morphine, the therapeutic effect on cancer pain is further enhanced, whereby the amount of administered morphine or the like can be reduced.

### Examples

The present invention will next be described in detail by way of the Examples and Comparative Example. The effect of compound (1) on cancer pain is described in Example 1 to Test Example 2. Preparation examples of the cancer pain therapeutic and/or prophylactic agent for use in the present invention are shown in Drug Preparation Examples 1 to 3.

### Example 1

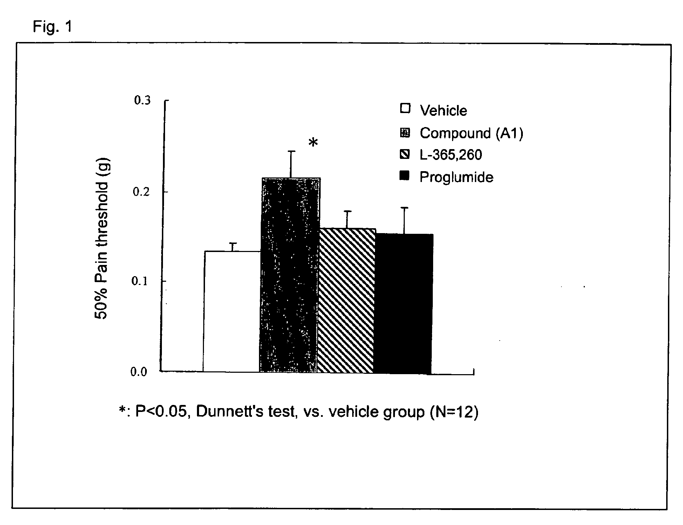

A B16-BL6 melanoma cell solution was subcutaneously injected into the plantar region of a right paw of a mouse by means of a syringe and injection needle, to thereby transplant melanoma cells into the mouse (2×10⁵ cells/mouse). After completion of cancer transplantation, the plantar region of the paw of each mouse was probed with von Frey filaments to thereby give contact stimulation to the paw, and pain threshold (load of filaments (g) required for withdrawal of the paw upon contact stimulation) was monitored. On day 14 after cancer transplantation, when the pain threshold considerably decreased to a constant value, a calcium salt of compound A (compound A1) was administered singly to the mouse. Then, the change in pain threshold was monitored. Compound (A1) was suspended in 0.5% CMC-Na solution before administration. Fig. 1 shows the results. In a cancer pain model, allodynia (i.e., pain caused by a contact stimulus that generally induces no pain) occurred, and the pain threshold was considerably lowered. Through peroral single administration of compound (A1) (100 mg/kg), the pain threshold was increased, to thereby improve allodynia. In contrast, peroral single administration of L-365,260 (CCK2 receptor antagonist) or proglumide (CCK2 receptor antagonist) (100 mg/kg) attained no effect on improvement of allodynia.

### Example 2

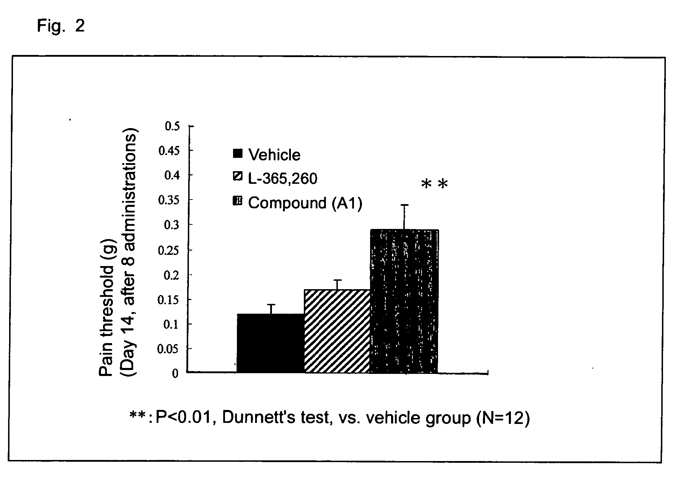

A B16-BL6 melanoma cell solution was subcutaneously injected into the plantar region of a right paw of a mouse by means of a syringe and injection needle, to thereby transplant melanoma cells into the mouse (2×10⁵ cells/mouse). After completion of cancer transplantation, the plantar region of the paw of each mouse was probed with von Frey filaments to thereby give contact stimulation to the paw, and pain threshold (load of filaments (g) required for withdrawal of the paw upon contact stimulation) was monitored. From day 7 after cancer transplantation, compound (A1) or L-365,260 (CCK2 receptor antagonist) (100 mg/kg) was orally administered once a day for eight days repeatedly, and the change in pain threshold was monitored. Fig. 2 shows the results. On day 7 after cancer transplantation, allodynia occurred, and a considerable drop in pain threshold occurred on day 14 after cancer transplantation. Peroral administration of compound (A1) elevated pain threshold, to thereby improve allodynia. In contrast, no effect on improvement of allodynia was observed in the case of administration of L-365,260.

### Example 3

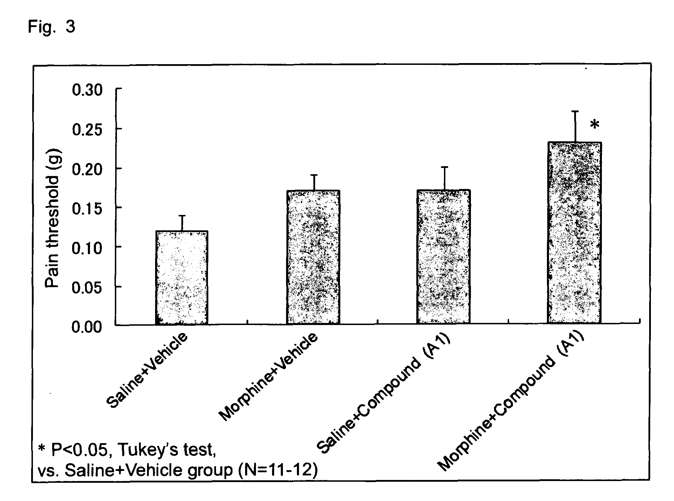

A B16-BL6 melanoma cell solution was subcutaneously injected into the plantar region of a right paw of a mouse by means of a syringe and injection needle, to thereby transplant melanoma cells into the mouse (2×10⁵ cells/mouse). After completion of cancer transplantation, the plantar region of the paw of each mouse was probed with von Frey filaments to thereby give contact stimulation to the paw, and pain threshold (load of filaments (g) required for withdrawal of the paw upon contact stimulation) was monitored. On day 14 after cancer transplantation, a compound A calcium salt (compound A1) (100 mg/kg) and morphine hydrochloride (2.5 mg/kg) were administered in combination. As a result, as shown in Fig. 3, the group of combined administration of compound A1 and morphine exhibited high anti-allodynia effect, as compared with the compound A1 sole administration group and the morphine sole administration group.

### Test Example 1

To pancreatic cancer patients who cannot receive resection, compound (A1) was orally administered. The test patients divided into three groups: a placebo group, a group of compound (A1) (120 mg) administration (120 mg × 2/day), and a group of compound (A1) (240 mg) administration (240 mg × 2/day). To all the test patients (23 cases), gemcitabine hydrochloride serving as a pancreatic cancer therapeutic agent was administered. In accordance with needs, analgesic agents including an opioid were administered. Percent pain improvement was found to be 12.5% in the placebo group, 57.0% in the compound (A1) (120 mg) group, and 37.5% in the compound (A1) (240 mg) group. Thus, the test has revealed that compound (A1) relieves the pain of pancreatic cancer patients.

### Test Example 2

In order to confirm whether or not the cancer pain relief effect observed in the compound (A1) administration group in the above clinical test is attributable to tumor regression effect, correlation between cancer pain scores and change in tumor diameter was investigated. Specifically, the differences in cancer pain score between before and after administration of compound (A1) and the changes in tumor diameter (longer diameter) were fitted through the least squares method, to thereby obtain a linear approximation formula and a correlation factor. The correlation factor (R²) was found to be 0.1044, indicating that there was less correlation between the cancer pain relief effect observed upon administration of compound (A1) and tumor regression effect. Therefore, the cancer pain relief effect of compound (A1) is thought to be not attributed to improvement in tissue damage surrounding cancer lesion provided by tumor regression effect.

### Drug Preparation Example 1

Compound (A1) (20 g), lactose (315 g), cornstarch (125 g), and crystalline cellulose (25 g) were uniformly mixed, and 7.5% aqueous hydroxypropyl cellulose solution (200 mL) was added to the mixture. The product was granulated by means of an extrusion granulator employing a screen (diameter: 0.5 mm), and the formed granules were immediately rounded by means of a Marumerizer and dried, to thereby provide a granule-form drug.

### Drug Preparation Example 2

Compound (A1) (20 g), lactose (100 g), cornstarch (36 g), crystalline cellulose (30 g), carboxymethyl cellulose calcium (10 g), and magnesium stearate (4 g) were uniformly mixed, and the mixture was pelletized by means of a one-shot pelletizer employing a frame (diameter: 7.5 mm), to thereby provide tablets (200 mg/tablet).

### Drug Preparation Example 3

Compound (A1) (100 mg), sodium acetate (2 mg), acetic acid (for adjusting pH to 5.8) (q.s.), and distilled water (balance) were mixed through a routine method to form an injection (10 mL/vial).

## Claims

1. A 1,5-benzodiazepine derivative represented by formula (1): 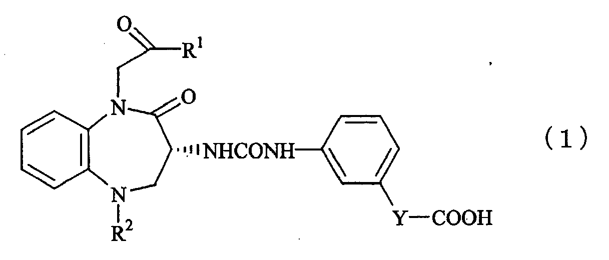 (wherein R¹ represents a C₁₋₆ alkyl group, R² represents a phenyl group or a cyclohexyl group, and Y represents a single bond or a C₁₋₄ alkylene group) or a pharmaceutically acceptable salt thereof, for use in the treatment or prevention of cancer pain at a peroral daily dose for an adult of 50 to 600 mg.

2. The 1,5-benzodiazepine derivative for use according to claim 1, wherein, in formula (1), R¹ is a tert-butyl group, R² is a cyclohexyl group, and Y is a single bond.

3. The 1,5-benzodiazepine derivative for use according to claim 1, wherein an active ingredient is (R)-(-)-3-[3-(1-tert-butylcarbonylmethyl-2-oxo-5-cyclohexyl-1,3,4,5-tetrahydro-2H-1,5-benzodiazepin-3-yl)ureido]benzoic acid or a pharmaceutically acceptable salt thereof.

4. The 1,5-benzodiazepine derivative for use according to claim 1, wherein an active ingredient is (R)-(-)-3-[3-(1-tert-butylcarbonylmethyl-2-oxo-5-cyclohexyl-1,3,4,5-tetrahydro-2H-1,5-benzodiazepin-3-yl)ureido]benzoic acid or a calcium salt thereof.

5. The 1,5-benzodiazepine derivative for use according to any one of claims 1 to 4, in the treatment or prevention of cancer pain at a peroral daily dose for an adult of 180 to 500 mg.

6. The 1,5-benzodiazepine derivative for use according to any one of claims 1 to 5 in combination with another analgesic agent.

## Patentansprüche

1. 1,5-Benzodiazepinderivat der Formel (1): 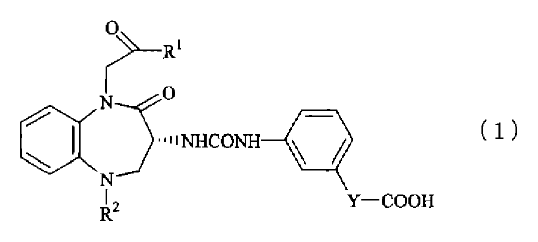 (wobei R¹ für eine C₁₋₆ Alkylgruppe steht, R² für eine Phenylgruppe oder eine Cyclohexylgruppe steht, und Y für eine Einfachbindung oder eine C₁₋₄Alkylengruppe steht) oder ein pharmazeutisch unbedenkliches Salz davon, zur Verwendung bei der Behandlung oder Prävention von Krebsschmerzen in einer peroralen Tagesdosis für einen Erwachsenen von 50 bis 600 mg.

2. Das 1,5-Benzodiazepinderivat zur Verwendung nach Anspruch 1, wobei in Formel (1) R¹ für eine tert-Butylgruppe steht, R² für eine Cyclohexylgruppe steht und Y für eine Einfachbindung steht.

3. Das 1,5-Benzodiazepinderivat zur Verwendung nach Anspruch 1, wobei ein wirksamer Bestandteil (R)-(-)-3-[3-(1 -tert-Butylcarbonylmethyl-2-oxo-5-cyclohexyl-1,3,4,5)-tetrahydro-2H-1,5-benzodiazepin-3-yl)ureido]benzoesäure oder ein pharmazeutisch unbedenkliches Salz davon ist.

4. Das 1,5-Benzodiazepinderivat zur Verwendung nach Anspruch 1, wobei ein wirksamer Bestandteil (R)-(-)-3-[3-(1 -tert-Butylcarbonylmethyl-2-oxo-5-Icyclohexyl-1,3,4,5-tetrahydro-2H-1,5-benzodiazepin-3-yl)ureido]benzoesäure oder ein Kalziumsalz davon ist.

5. Das 1,5-Benzodiazepinderivat nach einem der Ansprüche 1 bis 4, zur Verwendung bei der Behandlung oder Prävention von Krebsschmerzen in einer peroralen Tagesdosis für einen Erwachsenen von 180 bis 500 mg.

6. Das 1,5-Benzodiazepinderivat zur Verwendung nach einem der Ansprüche 1 bis 5 in Kombination mit einem weiteren analgetischen Mittel.

## Revendications

1. Dérivé de 1,5-benzodiazépine représenté par la formule (1) : 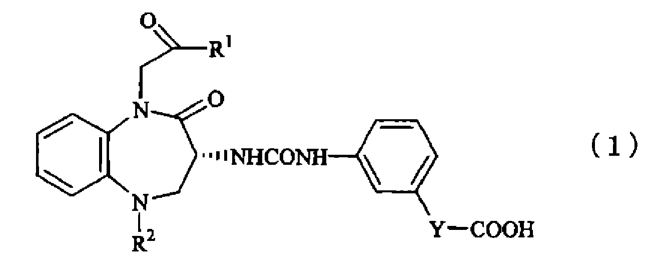 (dans laquelle R¹ représente un groupe alkyle en C₁ à C₆, R² représente un groupe phényle ou un groupe cyclohexyle, et Y représente une liaison simple ou un groupe alkylène en C₁ à C₄) ou un sel pharmaceutiquement acceptable de celui-ci, pour utilisation dans le traitement ou la prévention d'une douleur liée à un cancer à une dose quotidienne par voie orale pour un adulte de 50 à 600 mg.

2. Dérivé de 1,5-benzodiazépine pour utilisation selon la revendication 1, dans lequel, dans la formule (1) , R¹ est un groupe tert-butyle, R² est un groupe cyclohexyle, et Y est une liaison simple.

3. Dérivé de 1,5-benzodiazépine pour utilisation selon la revendication 1, dans lequel l'agent actif est placide (R)-(-)-3-[3-(1-tert-butylcarbonylméthyl-2-oxo-5-cyclohexyl-1,3,4,5-tétrahydro-2H-1,5-benzodiazépin-3-yl)-uréido]benzoïque ou un sel pharmaceutiquement acceptable de celui-ci.

4. Dérivé de 1,5-benzodiazépine pour utilisation selon la revendication 1, dans lequel l'agent actif est l'acide (R)-(-)-3-[3-(1-tert-butylcarbonylméthyl-2-oxo-5-cyclohexyl-1,3,4,5-tétrahydro-2H-1,5-benzodiazépin-3-yl)-uréido]benzoique ou un sel calcique de celui-ci.

5. Dérivé de 1,5-benzodiazépine pour utilisation selon l'une quelconque des revendications 1 à 4, dans le traitement ou la prévention d'une douleur liée à un cancer à une dose quotidienne par voie orale pour un adulte de 180 à 500 mg.

6. Dérivé de 1,5-benzodiazépine pour utilisation selon l'une quelconque des revendications 1 à 5, en combinaison avec un autre agent analgésique.
